# EUROPEAN PATENT SPECIFICATION

(11) **EP 2 707 062 B1**
(45) Date of publication and mention of the grant of the patent: **04.10.2017**
(21) Application number: 12719400.9
(22) Date of filing: 09.05.2012
(51) Int. Cl.: A61M 5/315

(54) **ASSEMBLY FOR A DRUG DELIVERY DEVICE**
ANORDNUNG FÜR EINE WIRKSTOFFFREISETZUNGSVORRICHTUNG
ENSEMBLE POUR DISPOSITIF D'ADMINISTRATION DE MÉDICAMENT

(30) Priority: 13.05.2011 US 201161485870 P
(43) Date of publication of application: 19.03.2014
(73) Proprietor: Sanofi-Aventis Deutschland GmbH, 65929 Frankfurt am Main (DE)
(72) Inventor: JUGL, Michael, 65926 Frankfurt am Main (DE); TEUCHER, Axel, 65926 Frankfurt am Main (DE)
(74) Representative: Keil & Schaafhausen Patent- und Rechtsanwälte PartGmbB
(86) International application number: PCT/EP2012/058569
(87) International publication number: WO 2012/156253

(56) References cited:
- EP-A1- 1 923 083
- WO-A1-01/95959
- WO-A1-2006/067480
- DE-A1- 3 144 825
- FR-A1- 2 586 192

## Description

### SUMMARY

The present application relates to drive mechanisms suitable for use in drug delivery devices, in particular pen-type injectors, wherein a number of settable or pre-set doses of medicinal product can be administered. In particular, the present application relates to injection devices that use a combination of friction wheels and linear bearing surfaces to set and deliver doses of medicament from a cartridge where an injection button elevates from an end of the device a distance proportional to the set dose and wherein the set dose is injected by pressing home the injection button. Mechanical advantage is supplied by the use of the friction wheels engaging the linear bearing surfaces.

### BACKGROUND

A number of pen-type injection devices are known to the art. Examples of such devices are described in published application WO 2008/058666 and in U.S. Pat. Nos. 7,241,278; 6,663,602; 5,743,889; and 6,889,698. In certain device designs, one or more gear wheels are used to engage stationary and moving racks or rods (i.e., a rack & pinion system) that move a piston rod axially during injection. The gear racks or rods move relative to each other, as a result of which the gear wheel placed between the racks experiences a correspondingly transmitted linear forward motion. The gear wheel or gear rod system is a form-locking connection that is susceptible to jamming. Any small amount of a foreign substance, such as dirt, lint, sand, or the like contaminants, can lodge in the toothed track of the rack/rods and cause the gear wheel/pinion to bind. Applying too strong of force in an attempt to un-jam the mechanism can cause warping of the mechanism. Such warping can prevent selecting the required dosage. In the case of further applications of pressure, the mechanism could be permanently damaged.

WO 01/95959 A1 discloses an injection device for injection of set doses of medicine from a cartridge, in which syringe a dose is set by screwing a nut up along a threaded piston rod whereby a dose setting drum, which carries on its cylindrical surface along a helix a scale of which a number corresponding to the set dose is shown in a window in the housing of the syringe, and an injection button, which is elevated over the end of the syringe, are moved axially a distance which is larger than the axial movement of the nut. A gear wheel gear transmission is established between the nut and the injection button. A further drive mechanism with a form-locking connection is disclosed in EP 1 923 083 A1.

WO 2006/067840 A1 disclosed a syringe drive comprising retaining means for retaining the body of a syringe, an exerting means for exerting a force to the syringe plunger, and sensing means for sensing the compressive force applied to the syringe plunger in use, wherein the sensing means comprises a pressure sensitive material, such as a quantum tunnelling material. Furthermore, it is disclosed that the exerting means may be a linear actor such as a geared worm screw driven by an electric motor, a friction wheel arrangement or a rack and pinion arrangement.

It is an object of the present disclosure provide an injection device that combines the advantages of the devices according to the prior art without adopting their disadvantages. These and other advantages will become evident from the following more detailed description.

### SUMMARY OF THE INVENTION

According to one aspect of the present application, a device or an assembly for a device is disclosed where there is established or provided a friction wheel or friction wheel assembly. The friction wheel or friction wheel assembly may be used to set and deliver doses of medicament. In one embodiment, the friction wheel or friction wheel assembly can transform rotational movement to linear movement between a dose setting drum and a piston rod. The combined use of a friction wheel with a linear bearing surface eliminates the known rack and pinion assembly where one or more toothed gear wheel engages corresponding teeth on racks or other linear surfaces.

The drug delivery device, preferably an injection device, according to the present application replaces the gear wheels and associated racks or rods of prior art devices and instead uses a friction wheel system or assembly to provide the linear motion needed to set a dose and to drive the piston rod in an axial distal direction during dose delivery. The force exerted by a user on pushing the dose button on the injection device may transmit power to one or more friction wheels that are engaged and drive one or more linear bearing surfaces, i.e., the "friction wheel system or assembly." The piston rod may be directly or indirectly connected to one of these bearing surfaces and the movement of bearing surface may drive the piston rod. In some embodiments, the piston rod itself may comprise the bearing surface as an integral component. The counter bearing may serve to guide the friction wheel.

Examples of embodiments for the friction wheel may include, but are not limited to, metal body wheels coated with an elastomer (hardness up to 80 ShoreA) or a rubber ring (elastomer) is pulled onto a round base body (metal or plastic).

Some of the advantages of using a friction wheel assembly include that in case of a jam of the threaded piston rod, the frictional lock may be overcome starting at a defined force (sliding coupling) and a low noise level because there is no meshing of gear teeth with rack teeth. Furthermore no jamming occurs because there are no teeth and no potential damage to the mechanism. The friction wheel may be "insensitive to tolerances", i.e., in a gearing based device, the division of gears is subject to the corresponding tolerances. This may have a direct effect on the uniformity of the feed motion. A narrowing of the tolerances of the gears may lead to an increase in manufacturing costs. Due to availability of various types of elastomers, the hardness and the "sliding coupling" may be adjusted, i.e. the force for sliding through may be set, for example, on a country specific basis.

The "distal end" of the device or a component of the device shall mean the end, which is closest to the dispensing end of the device. The "proximal end" of the device or a component of the device shall mean the end, which is furthest away from the dispensing end of the device.

According to a first aspect of the present application, a drive mechanism for use in a drug delivery device is provided comprising a housing having a proximal and a distal end, a drive member located within the said housing such that the said drive member is movable longitudinally. Furthermore, a piston rod adapted to operate through the housing and transfer a force in the longitudinal direction to the distal end of the drug delivery device may be provided. The drive mechanism may further comprise a rotating friction wheel releasably engaged with the said piston rod and engaged to the said drive member and engaged to the said housing. This drive mechanism is characterized in that when the drive member moves proximally with respect to the housing the friction wheel may move proximally with respect to the piston rod. When the drive member moves distally the friction wheel may engage a linear bearing surface as it rotates and distally displaces the piston rod towards the distal end of the device. In a preferred embodiment of the drive mechanism the drive member is non-rotatable with respect to the housing. In another preferred embodiment the piston rod is non-rotatable with respect to the housing. In a further preferred embodiment the engagement between the friction wheel and the piston rod acts through an axle of the friction wheel and preferably through a carrier plate supporting the friction wheel. In yet another further preferred embodiment of the drive mechanism the friction wheel is designed for engagement with a first linear bearing surface located on the drive member and a second linear bearing surface located on the housing. Most preferably, the first bearing surface is axially slidable relative to the housing and the second bearing surface is fixed relative to the housing.

In a preferred embodiment the friction wheel is connected to a carrier plate having pawl arms. The engagement between the piston rod and the friction wheel may act through an axle.

In another embodiment the injection device comprises a housing wherein a piston rod, threaded with a first pitch, is non rotatable, but longitudinally displaceable, having a nut engaging the thread of the piston rod, which nut can be screwed along the threaded piston rod away from a defined position in the housing to set a dose and can be pressed back to the defined position carrying the piston rod with it when the set dose is injected. There is a dose setting drum which can be screwed outward away from the housing in the proximal direction along a thread having a second pitch to lift an injection button with it from the proximal end of the housing. This injection device also includes a friction wheel assembly that provides a mechanical advantage between the axial movements of the dose setting drum and/or the injection button and the nut relative to the housing, where the ratio of mechanical advantage is equal to the ratio of the second pitch to the first pitch. The first pitch can be less than or equal to the second pitch.

In another embodiment of the application the injection device may comprise a housing wherein a piston rod, threaded with a first pitch, is non rotatable, but longitudinally displaceable with respect to the housing. Furthermore, the injection device may comprise a displaceable nut. The nut may move relative to the housing and may engage the thread of the piston rod The nut may be configured to be screwed along the threaded piston rod away from a defined position in the housing to set a dose and may be pressed back to the defined position carrying the piston rod with it when the set dose is injected. In particular, the nut may be capable of moving along the piston rod in proximal direction from a first position on the piston rod to a second position on the piston rod. The displacement of the nut along the piston rod in a proximal direction may define a quantity of medication to be injected by the injection device. The injection device may comprise a dose setting drum. The dose setting drum may comprise a threaded surface with a second pitch. Furthermore, the dose setting drum may comprise an injection button being disposed on an end thereof. The dose setting drum may be configured to engage the housing and may be rotatable within the housing, such that it may be screwed outward away from the housing in the proximal direction along the thread having the second pitch to lift the injection button with it from the proximal end of the housing. This injection device may also include a friction wheel assembly. The friction wheel assembly may be configured for coupling axial movement of the injection button with axial movement of the nut. Furthermore, the friction wheel assembly may provide a mechanical advantage between the axial movements of the dose setting drum and the injection button and the nut relative to the housing. The ratio of mechanical advantage may be equal to the ratio of the second pitch to the first pitch. The first pitch may be less than or equal to the second pitch.

In a preferred embodiment, the mechanical advantage between the movements of the injection button and the dose setting drum and the nut may be obtained by the friction wheel assembly comprising at least one friction wheel carried by a connector which projects from the external surface of the nut, where the connector is longitudinally displaceable but non-rotatable relative to the nut, a first bearing surface integral with a first element of the friction wheel assembly, which element is rotational but not longitudinally displaceable relative to the housing, and a second element of the friction wheel assembly carrying a second bearing surface projecting from said friction wheel assembly longitudinally displaceable, but non-rotatable relative to the first element and being coupled to the injection button to follow longitudinal movements of the button and the dose setting drum. The at least one friction wheel engages the first and the second linear bearing surfaces, respectively, and being dimensioned to provide a mechanical advantage by which a longitudinal movement of the second bearing surface is transformed to a longitudinal movement of the connector with a ratio for the mentioned longitudinal movements of the second bearing surface and the connector relative to the housing that equals the ratio of the second pitch to the first pitch. The ratio of the second pitch to the first pitch may be 2:1.

The piston rod may be provided with a stop. The stop may be configured to engage and halt the movement of the nut along the thread of the piston rod when the amount of medicament remaining in the container or cartridge equals the set dose. This stop provides a classical dose setting limiter that involves no additional members to prevent setting of a dose exceeding the amount of liquid left in the cartridge or ampoule containing a medicament.

These as well as other advantages of various aspects of the present application will become apparent to those of ordinary skill in the art by reading the following detailed description, with appropriate reference to the accompanying figures. The invention is defined by the subject-matter of claim 1.

The term "medicament" or medication, as used herein, preferably means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl-ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 Exendin-4(1-39),
des Pro36 [Asp28] Exendin-4(1-39),
des Pro36 [IsoAsp28] Exendin-4(1-39),
des Pro36 [Met(0)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(02)25, IsoAsp28] Exendin-4(1-39); or
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39),
wherein the group -Lys6-NH2 may be bound to the C-terminus of the Exendin-4 derivative;
or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(O2)25, Asp28] Exendin-4(1-39)-Lys6-NH2,
H-des Asp28 Pro36, Pro37, Pro38 [Trp(02)25] Exendin-4(1-39)-NH2,
H-(Lys)6-des Pro36, Pro37, Pro38 [Trp(O2)25, Asp28] Exendin-4(1-39)-NH2,
H-Asn-(Glu)5-des Pro36, Pro37, Pro38 [Trp(O2)25, Asp28] Exendin-4(1-39)-NH2,
des Pro36, Pro37, Pro38 [Trp(O2)25, Asp28] Exendin-4(1-39)-(Lys)6-NH2,
H-(Lys)6-des Pro36, Pro37, Pro38 [Trp(O2)25, Asp28] Exendin-4(1-39)-(Lys)6-NH2,
H-Asn-(Glu)5-des Pro36, Pro37, Pro38 [Trp(O2)25, Asp28] Exendin-4(1-39)-(Lys)6-NH2,
H-(Lys)6-des Pro36 [Met(0)14, Asp28] Exendin-4(1-39)-Lys6-NH2,
des Met(0)14 Asp28 Pro36, Pro37, Pro38 Exendin-4(1-39)-NH2,
H-(Lys)6-desPro36, Pro37, Pro38 [Met(0)14, Asp28] Exendin-4(1-39)-NH2,
H-Asn-(Glu)5-des Pro36, Pro37, Pro38 [Met(0)14, Asp28] Exendin-4(1-39)-NH2,
des Pro36, Pro37, Pro38 [Met(0)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(0)14, Asp28] Exendin-4(1-39)-(Lys)6-NH2,
H-Lys6-des Pro36 [Met(0)14, Trp(O2)25, Asp28] Exendin-4(1-39)-Lys6-NH2,
H-des Asp28 Pro36, Pro37, Pro38 [Met(0)14, Trp(O2)25] Exendin-4(1-39)-NH2,
H-(Lys)6-des Pro36, Pro37, Pro38 [Met(0)14, Asp28] Exendin-4(1-39)-NH2,
H-Asn-(Glu)5-des Pro36, Pro37, Pro38 [Met(0)14, Trp(O2)25, Asp28] Exendin-4(1-39)-NH2,
des Pro36, Pro37, Pro38 [Met(0)14, Trp(O2)25, Asp28] Exendin-4(1-39)-(Lys)6-NH2,
H-(Lys)6-des Pro36, Pro37, Pro38 [Met(O)14, Trp(O2)25, Asp28] Exendin-4(S1-39)-(Lys)6-NH2,
H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (∼150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

### BRIEF DESCRIPTION OF THE FIGURES

In the following the present application is described in further details with references to the figures, wherein
FIG. 1 schematically shows a sectional view of an injection device according to the present application;
FIG. 2 shows schematically a sectional view of the friction wheel assembly along the line II in FIG. 1;
FIG. 3 shows a longitudinal sectional view in the dose setting part of another embodiment of an injection device;
FIG. 4 shows a longitudinal sectional view perpendicular to the view in FIG. 3;

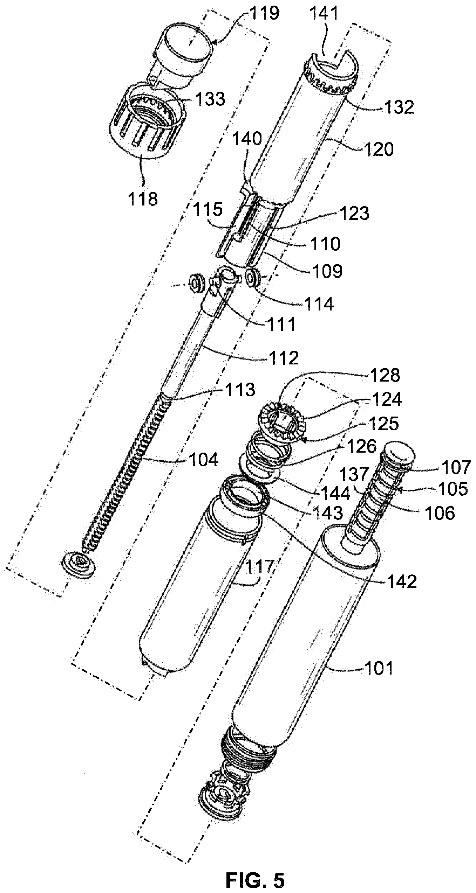

FIG. 5 shows an exploded picture of the of the device shown in FIGS. 3 and 4;
FIG. 6 shows a sectional view of another embodiment of the drug delivery device in accordance with the present invention in a first, cartridge full, position; and
FIG. 7 shows a further sectional view of the embodiment of the drug delivery device of FIG. 6.

### DETAILED DESCRIPTION OF PREFERRED EMBODIMENTS

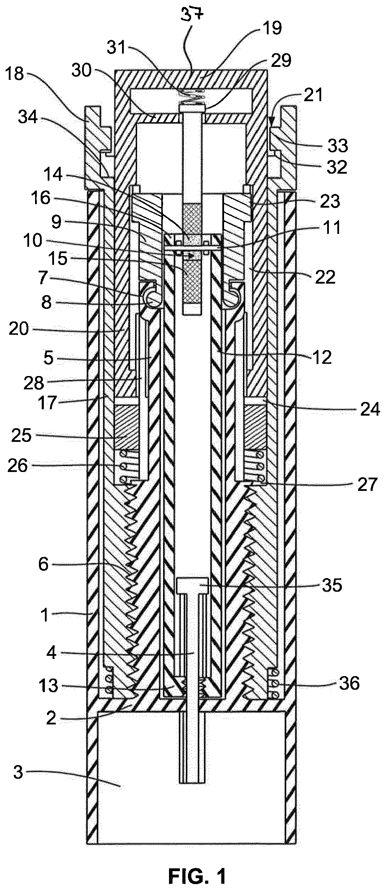

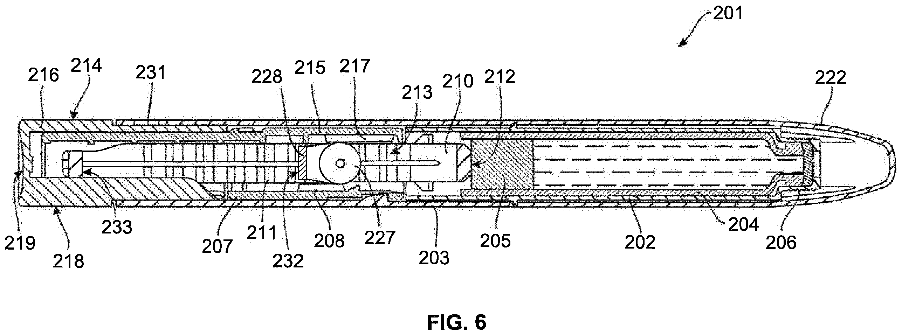

In the device shown in FIG. 1 an elongated cylindrical housing 1 has a partitioning wall 2 that divides the housing into a compartment containing a dose setting mechanism and a compartment 3 configured to accommodate a not shown ampoule. An example of such a cartridge or ampoule is illustrated as item 204 in another embodiment of the injection device shown in Fig. 6. A threaded piston rod 4 has a not round cross section by which it fits through a central opening in the wall 2 so that the piston rod 4 can be displaced longitudinally through the central opening in the wall 2 but not rotated relative to this wall.

Concentrically with the housing 1 the wall 2 carries on its side turning away from the compartment 3 a tubular element 5 a part of which is adjacent to the wall 2 provided with an outer thread 6 and which has at its free end a circumferential recess 7. A ring shaped coupling element 8 on a friction wheel assembly 9 engages the recess 7. By this coupling the friction wheel assembly is fixed in the housing 1 in a way that allows the friction wheel assembly 9 to rotate in the housing 1 but not to be axially displaced relative to said housing 1.

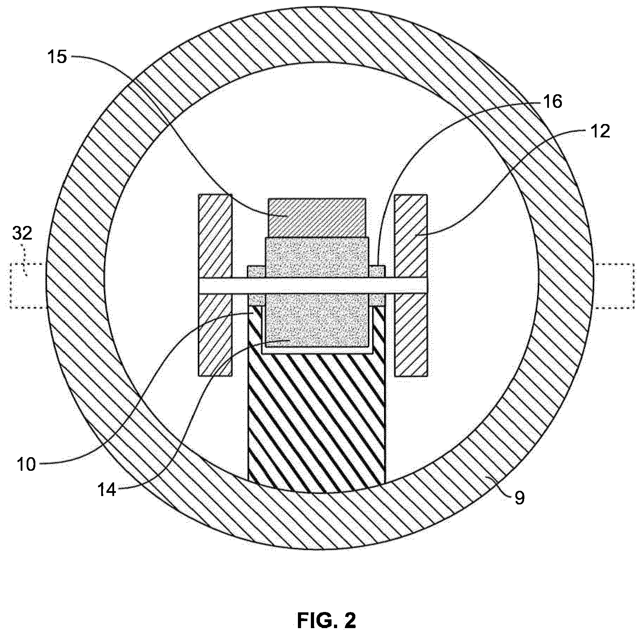

In the friction wheel assembly 9 there can be two integral friction wheels journaled on a shaft 11, which runs perpendicular to the longitudinal axis of the device between two axial connection bars 12. The connection bars 12 project from the friction wheel assembly towards the partition wall 2 and are connected to a nut 13, which adjacent to the wall 2 engages the thread of the piston rod 4. The friction wheel assembly 9 comprises a friction wheel 14 that frictionally engages a linear bearing surface 15, which is guided in the friction wheel assembly 9 to be displaced in the longitudinal direction of the device, and a friction wheel 16 with a smaller diameter engaging a bearing surface 10 in FIG. 2 extending in the longitudinal direction of the device on the inner wall of the friction wheel assembly 9. The friction wheel 16 with the smaller diameter may be divided into two friction wheels placed on each side of the of friction wheel 14.

A tubular dose setting drum 17 fitting into the housing 2 is at an end provided with an internal thread mating and engaging the outer thread 6 of the tubular element 5 and has at its other end a part with enlarged diameter forming a dose setting button 18. Due to the engagement with the thread 6 the dose setting drum 17 may be screwed in and out of the housing 1 to show a number on a not shown helical scale on its outer surface in a not shown window in the housing 1.

A bottom 19 in a deep cup shaped element, which has a tubular part 20 fitting into the dose setting drum 17 and encompassing the friction wheel assembly 9, forms an injection button. Coupling means between the dose setting drum 17 and the cup shaped element ensures that rotation of the dose setting drum 17 is transmitted to the cup shaped element. Further, the inner wall of the tubular part 20 has longitudinal recesses 22 engaged by protrusions 23 on the friction wheel assembly 9 so that rotation of the dose setting drum 17 via the cup shaped element is transmitted to the friction wheel assembly 9.

At the edge of the open end of the cup shaped element a rosette of V-shaped teeth are provided, which teeth engage a corresponding rosette of V-shaped teeth 24 on a ring 25 which is pressed against the edge of the cup shaped element by a spring 26, which is compressed between a non-toothed side of the ring 25 and a round track on shoulder 27 on the inner wall of the dose setting drum 17 at an inner end of the inner thread of this drum. The ring 25 is provided with an inner recess, which is engaged by a longitudinal rib 28 on the tubular element 5 so that the ring 25 can be displaced in the axial direction of the device, but cannot be rotated relative to the housing 1. Thereby, a click coupling is established which makes a click noise when the V-shaped teeth at the edge of the cup shaped element by rotation of this element rides over the V-shaped teeth of the ring 25.

A head 29 on the projecting end of the bearing surface 15 is fixed at the bottom of the cup shaped element between the bottom 19 forming the injection button 37 and an inner wall 30 near this bottom. The bearing surface is fixed in a position with its head pressed against the wall 30 by a spring 31 between the bottom 19 and the head 29.

To set a dose, the dose setting button 18 is rotated to screw the dose setting drum 17 up along the thread 6. Due to the coupling 21 the cup shaped element will follow the rotation of the dose setting drum 17 and it will be lifted with this drum up from the end of the housing 1. By the rotation of the cup shaped element the V-shaped teeth 24 at the edge of its open end will ride over the V-shaped teeth of the non-rotatable ring 25 to make a click sound for each unit the dose is changed. If too high a dose is set, it can be reduced by rotating the dose setting button 18 in the opposite direction of the direction for increasing the dose. When the dose setting drum is screwed up along the thread 6 on the tubular element 5 the ring 25 will follow the dose setting drum in its axial movement as the spring 26 is supported on the shoulder 27. The spring will keep the V-shaped teeth of the ring 25 and the cup shaped element in engagement and maintain in engagement the coupling 21, which may comprise an inverted V-shaped protrusions 32 on the cup shaped element engaging V-shaped recesses in an inner ring 33 in the dose setting button 18.

The rotation of the dose setting button 18 and the cup shaped element is further transmitted to the friction wheel assembly 9 through the protrusions 23 on this friction wheel assembly engaging the longitudinal recesses 22 in the inner wall of the tubular part 20 of the cup shaped element. The rotation of the friction wheel assembly 9 is through the connection bars 12 transmitted to the nut 13, which is this way screwed up along the thread of the piston rod 4 and lifted away from its abutment with the wall 2 when a dose it set. As the dose is set by moving the nut 13 on the piston rod that operates a piston in a cartridge or ampoule of medicament (not shown) in the compartment 3, a dose setting limiter, which ensures that the size of the set dose does not exceed the amount of medicament left in the ampoule, can be established by providing the piston rod 4 with a stop 35 which limits the movement of the nut 13 up along the piston rod 4.

Due to the confinement of the head 29 in the space between the bottom 19 and the wall 30 of the cup shaped element, the bearing surface 15 is drawn with the injection button 37 outward. Also the axial movement of the nut 13 relative to the housing 1 will be transmitted to the friction wheel assembly 9 through the connection bars 12 and this movement will through the friction wheel assembly 9 induce an outward movement of the bearing surface 15. This induced outward movement has to be the same as the outward movement induced by outward movement of the injection button 37. This is obtained by dimensioning the diameter of the friction wheels contained in the friction wheel assembly 9 so that the ratio of the movements of the connection bars 12 and the bearing surface 15 relative to the housing corresponds to the ratio of the pitches for the thread on the piston rod and for the thread 6 for the longitudinal movement of the dose setting drum 17. The friction force between the friction wheel and bearing surface 15 is adjusted by varying the roughness of the bearing surface 15, the friction wheel surface, the materials of construction of the bearing surface 15 or friction wheels, or both. Texturing the surfaces or using different materials of construction can perform this manipulation of the roughness.

To inject a set dose, the injection button 37 is pressed by pressing on the bottom 19. In the initial phase of the pressing the button 37, the spring 31 is compressed where after the pressing force is directly transmitted to the head 29 of the bearing surface 15 and this way to the bearing surface 15 itself. Through the friction wheel assembly 9, the force is transformed and is transmitted through the connection bars 12 to the nut 13, which will press the piston rod 4 into the compartment 3 until the dose-setting drum 17 abuts the wall 2.

During the initial phase of the movement of the injection button 37 the inverted V-shaped protrusions 32 on the cup shaped element will be drawn out of their engagement with the V-shaped recesses in the ring 33. The dose setting drum 17 can now rotate relative to the injection button and will do so when the inverted V-shaped protrusions 32 press against a shoulder 34 at the bottom of the dose setting button 18. Only a force sufficient to make the dose setting drum rotate to screw itself downward along the thread 6 is necessary as the force necessary to make the injection is transmitted to the piston rod 4 through the friction wheel assembly 9. A helical reset spring 36 concentric with the dose setting drum can be mounted at the lower end of this drum and can have one end anchored in the dose setting drum 17 and the other end anchored in the wall 2. During setting of a dose this spring may be tighter coiled so that on the dose setting drum 17 it exerts a torque approximately corresponding to the torque necessary to overcome the friction in the movement of the dose setting drum 17 along the thread 6 so that the force which the user have to exert on the injection button 37 is only the force necessary to drive the piston rod into an ampoule to inject the set dose.

It shall be noticed that use of only one size friction wheel that engages the bearing surface 15, which is movable relative to the friction wheel assembly 9, as the bearing surface 10, which is unmovable relative to the friction wheel assembly 9, provides a friction wheel ratio or mechanical advantage of 2:1 for the longitudinal movement relative to the syringe housing 1 for the movable bearing surface 15 and the connector 12, which carries the shaft 11 of the friction wheel 14.

In the device as described with reference to Figure 1, the connection bars 12 are fixed relative to the nut 13. Yet, in another embodiment, the connection bars may be, e.g. longitudinally, displaceable with respect to the nut 13.

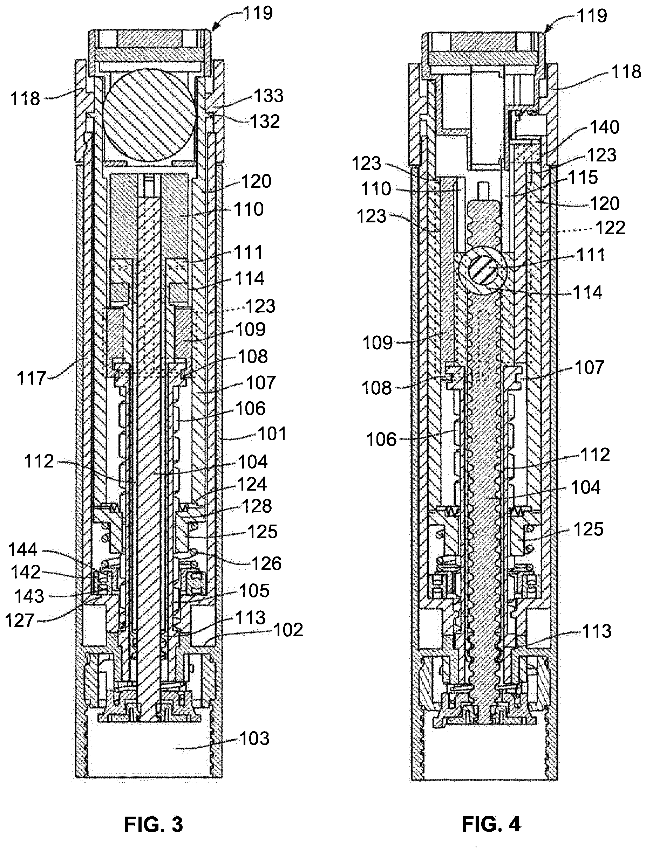

FIGS. 3 and 4 show another preferred embodiment wherein only one size friction wheel is used and wherein elements corresponding to elements in FIGS. 1 and 2 are given the same references as these elements with a prefixed "1". The partitioning wall 102 and the tubular element 105 are made as two parts which are by the assembling of the device connected to each other to make the assembled parts act as one integral part. The same way the dose setting drum 117 and the dose setting button 118 are made as two parts, which are fixed firmly together.

A circumferential recess 107 is provided as an outer recess at the free end of the tubular part 105 and a ring shaped coupling element is provided as an inner bead 108 on the friction wheel assembly element 109. The bead 108 engages the recess 107 to provide a rotatable but not axially displaceable connection between the tubular part 105 and the friction wheel assembly.

A tubular element 120 having ridges 122 which engages recesses 123 on the friction wheel assembly is at its upper end closed by a button 119. Pressing this button 119 creates a force that is transmitted to the tubular element 120.

The friction wheel assembly is formed by two shells, which together form a cylinder fitting into the tubular element where the shells are guided by the engagement between the ridges 122 and the recesses 123. Bearing surfaces 110 and 115 are provided along edges of the shells facing each other. One shell forming the friction wheel assembly element 109 is provided with the inner bead 108, which engages the circumferential recess 107 at the end of the central tubular part 105 and carries the bearing surface 110. The other shell is axially displaceable in the tubular element 120 and forms the bearing surface 115. At its outer end projecting from the friction wheel assembly the shell carrying the bearing surface 115 is provided with a flange 140 that is positioned in a cut out 141 in the end of the tubular element 120 carrying the button 119 so that this button and the tubular element 120 can be moved so far inward in the device that the engagement of the teeth 132 and 133 can be released before the button 119 abuts the flange 140.

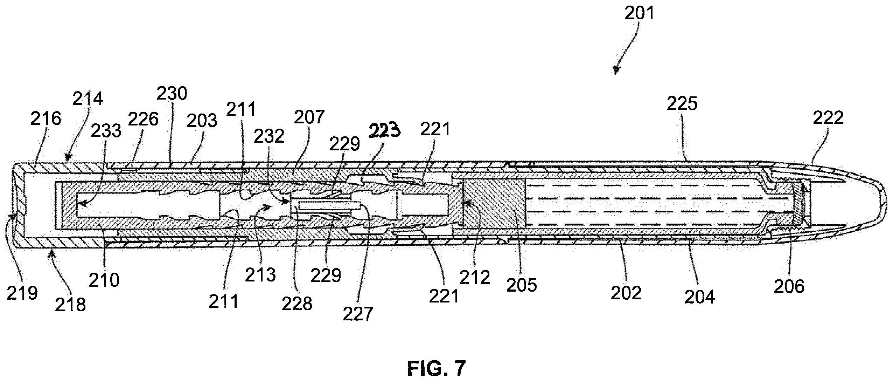

A tubular connection element 112 connects the threaded piston rod 104 with the friction wheel assembly. At the end of connection element 112 is a nut 113 that engages the piston rod 104. Nut 113 has an internal thread mating the external thread of the piston rod. At the opposite end of the connection element 112 are two pins 111 projecting perpendicular to the longitudinal axis of the connection element 112 at each side of this element. This end of the connection element engages the friction wheel assembly. Each pin 111 carries a friction wheel 114 that is placed between and engages the two bearing surfaces 110 and 115. This way the connection element 112 will be rotated with the friction wheel assembly but can be displaced axially relative to said friction wheel assembly when the bearing surfaces 110 and 115 are moved relative to each other. In practice it will be the bearing surface 115, which is moved relative to the friction wheel assembly element 109 and the housing and will result in a movement of the connection element 112 relative to housing a distance that is half the distance that the bearing surface 115 is moved, provided the diameters of the friction wheels are chosen to result in a mechanical advantage is 2:1. A ring 125 which is at its periphery provided with a rosette of teeth 124 and has a central bore fitting over the central tube in the housing 101 so that this ring 125 can be axially displaced along said central tube 105, but internal ridges 128 in the central bore of the ring 125 engages longitudinal recesses 137 in the central tube to make the ring non-rotatable in the housing so that a rosette of teeth at the edge of the tubular element 120 can click over the teeth 124 of the ring when said tubular element is rotated together with the dose setting drum 117. A spring 126 working between the ring 125 and an internal shoulder 127 provided in the dose setting drum 117 makes the ring follow the tubular element 120 when this element with the dose setting drum is moved longitudinally in the housing. To make the dose setting drum easy to rotate, especially when said dose setting drum is pressed inward in the housing, a roller bearing is included having an outer ring 142 supported by the shoulder 127 and an inner ring 143 supporting a pressure bushing 144 which supports the spring 126. By the provision of this smooth running support only very small axial forces are needed to rotate the dose setting drum 117 back to its zero position when a set dose is injected. This solution replaces the provision of a reset spring as the spring 36 in FIG. 1. The bearing is shown as a radial bearing but can be replaced by an axial bearing. Referring next to FIGS. 6 & 7, there is shown a drug delivery device in accordance with the present invention. The drug delivery device 201 comprises a cartridge retaining part 202, and a main (exterior) housing part 203. The proximal end of the cartridge retaining part 202 and the distal end of the main housing 203 are secured together by any suitable means known to the person skilled in the art. In the illustrated embodiment, the cartridge retaining part 202 is secured within the distal end of the main housing part 203.

A cartridge 204 from which a number of doses of a medicinal product may be dispensed is provided in the cartridge retaining part 202. A piston 205 is retained in the proximal end of the cartridge 204. A removable cap 222 is releasably retained over the distal end of the cartridge retaining part 202. The removable cap 222 may be optionally provided with one or more window apertures through which the position of the piston 205 within the cartridge 204 can be viewed. The distal end of the cartridge retaining part 202 in the illustrated embodiment, is provided with a distal threaded region 206 designed for the attachment of a suitable needle assembly to enable medicament to be dispensed from the cartridge 204.

In the illustrated embodiment, the main housing part 203 is provided with an internal housing 207. The internal housing 207 is secured against rotational and/or axial movement with respect to the main housing part 203. The internal housing is provided with a first linear bearing surface 208 extending along the main axis of the internal housing 207. Alternatively, the internal housing 207 may be formed integrally with the main housing part 203. Additionally, the internal housing 207 is provided with a plurality of guide lugs (not shown) and pawl means (not shown). The pawl means may be an integral part of the internal housing 207 or may be a separate component as illustrated.

A piston rod 210 extending through the main housing 203 has a first set of indentations (not shown) extending longitudinally along external surfaces of the piston rod 210. A second set of indentations 211 extend longitudinally along internal surfaces of the piston rod 210. The first set of indentations (element 223 in Fig. 7) of the piston rod 210 extend through and are engaged with the pawl means (element 221 in Fig. 7) of the internal housing 207 to prevent movement of the piston rod 210 in the proximal direction during setting of the device. A bearing surface 212 located at the distal end of the piston rod 210 is disposed to abut the proximal face of the piston 205. In the illustrated embodiment the longitudinal spacing of the first set of indentations and the second set of indentations 211 is essentially equal.

A friction wheel assembly 213, consisting of a carrier 228 and a friction wheel 227, free to rotate within the carrier 228, is located within a channel within the piston rod 210. Pawl arms 229 located on the carrier 228 are releasably engaged with the second set of indentations 211 of the piston rod 210. The pawl arms 229 of the carrier 228 are designed to transmit force to the piston rod 210 in the distal direction during dispense and to allow relative movement between the friction wheel assembly 213 and the piston rod 210 in the proximal direction during setting. The circumferential surface of the friction wheel 227 is permanently engaged with a first the linear bearing surface 208 of the internal housing 207.

A drive member 214 extends about the piston rod 210. The drive member 214 comprises a longitudinal part 215 and an activation part 216. The longitudinal part 215 and the activation part 216 are secured to each other to prevent rotational and/or axial movement there between. Alternatively, the drive member 214 may be a unitary component consisting of a longitudinal part 215 and activation part 216. The longitudinal part 215 is provided with a second linear bearing surface 217 extending along the main axis of the longitudinal part 215. The second linear bearing surface 217 is permanently engaged with the circumferential surface of the friction wheel 227.

The drive member 214 has a plurality of guide slots (not shown) in which the guide lugs (not shown) of the internal housing 207 are located. These guide slots define the extent of permissible axial movement of the drive member 214 with respect to the housing part 203. In the illustrated embodiment the guide slots also prevent rotational movement of the drive member 214 relative to the main housing part 203. The activation part 216 of the drive member 214 has a plurality of grip surfaces 218 and a dispensing face 219.

To increase intuitiveness of the operation of the device, the main housing part 203 may optionally be provided with a window aperture through which graphical status indicators provided on the drive member 214, can be viewed.

Operation of the drug delivery device in accordance with the present invention will now be described. To set a dose a user grips the grip surfaces 218 of the drive member 214. The user then pulls the drive member 214 in a proximal direction away from the main housing part 203 thereby moving the longitudinal part 215 in a proximal direction. The proximal movement of the longitudinal part 215 causes the friction wheel 227 to rotate and move proximally by virtue of the engagement with the circumferential surface of the friction wheel 227 with the second linear bearing surface 217 of the longitudinal part 215 and the first linear bearing surface 208 of the internal housing 207 thus moving the friction wheel assembly 213 in the proximal direction.

The piston rod 210 is prevented from moving proximally by interaction of pawl means 221 of the internal housing 207 with a first set of indentations 223 on the piston rod 210. As the drive member 214 travels in the proximal direction relative to the piston rod 210, the pawl arms 229 of the carrier 228 are displaced inwardly by interaction with the second set of indentations 211 of the piston rod 210.

The proximal travel of the drive member 214 is limited by the guide slots of the longitudinal part 215. At the end of the travel of the drive member 214, the pawl arms 229 of the carrier 228 engage with the next sequential indentation of the second set of indentations 211 of the piston rod 210. The action of the pawl arms 229 of the carrier 228 positively engaging the second set of indentations 211 of the piston rod 210 creates an audible and tactile feedback to the user to indicate that the dose has been set. Additionally, visual feedback regarding dose setting may optionally be indicated by a graphical status indicator provided on the drive member 214, which can be viewed through an optional window aperture in the main housing part 203.
When the dose has been set, the user may then dispense this dose by depressing the dispensing face 219 of the activation part 216 of the drive member 214. By this action the drive member 214 and the longitudinal part 215 are moved axially in the distal direction relative to the main housing part 203. As the circumferential surface of the friction wheel 213 is engaged with second linear bearing surface 217 of the longitudinal part 215 and the first linear bearing surface 208 of the internal housing 207, the friction wheel 227 is caused to rotate and move in the distal direction thus moving the friction wheel assembly 213 longitudinally in the distal direction. As the pawl arms 229 of the carrier 228 of the friction wheel assembly 213 is engaged with the second set of indentations 211 of the piston rod 210, the piston rod 210 is caused to move longitudinally in the distal direction with respect to the internal housing 207.

The distal axial movement of the piston rod 210 causes the bearing surface 212 of the piston rod 210 to bear against the piston 205 of the cartridge 204 causing a dose of medicament to be dispensed through the attached needle (not shown). The distal travel of the drive member 214 is limited by the guide slots (not shown) of the longitudinal part 215. Audible and tactile feedback to indicate that the dose has been dispensed is provided by the interaction of the pawl means (not shown) of the internal housing 207 with the first set of indentations (not shown) of the piston rod 210. Additionally, visual feedback regarding dose dispensing may optionally be indicated by a graphical status indicator, provided on the drive member 214, which can be viewed through an optional window aperture in the main housing part 203.

Further doses may be delivered as required up to a pre-determined maximum number of doses. When the maximum number of doses has been delivered the proximal face 232 of the carrier 228 abuts an internal distal face 233 of the piston rod 210 to prevent further axial movement of the gear 213 and thus the drive member 214 in proximal direction. Exemplary embodiments of the present invention have been described. Those skilled in the art will understand, however, that changes and modifications may be made to these embodiments without departing from the scope of the present invention, which is defined by the claims.

### Reference numerals

- 1: housing
- 2: wall
- 3: compartment
- 4: piston rod
- 5: tubular element
- 6: thread
- 7: recess
- 8: coupling element
- 9: friction wheel assembly
- 10: bearing surface
- 11: shaft
- 12: connection bars
- 13: nut
- 14: friction wheel
- 15: bearing surface
- 16: friction wheel
- 17: dose setting drum
- 18: dose setting button
- 19: bottom
- 20: tubular part
- 21: coupling
- 22: longitudinal recesses
- 23: protrusions
- 24: V-shaped teeth
- 25: ring
- 26: spring
- 27: shoulder
- 28: longitudinal rib
- 29: head
- 30: wall
- 31: spring
- 32: protrusions
- 33: ring
- 34: shoulder
- 35: stop
- 36: spring
- 37: injection button
- 101: housing
- 102: wall
- 103: compartment
- 104: piston rod
- 105: tubular element
- 106: thread
- 107: recess
- 108: bead
- 109: friction wheel assembly
- 110: bearing surface
- 111: pins
- 112: connection element
- 113: nut
- 114: friction wheel
- 115: bearing surface
- 117: dose setting drum
- 118: dose setting button
- 119: button
- 120: tubular element
- 122: ridges
- 123: recesses
- 124: teeth
- 125: ring
- 126: spring
- 127: shoulder
- 128: ridges
- 132: teeth
- 133: teeth
- 137: recesses
- 140: flange
- 141: cut out
- 142: outer ring
- 143: inner ring
- 144: bushing
- 201: drug delivery device
- 202: cartridge retaining part
- 203: main housing
- 204: cartridge
- 205: piston
- 206: threaded region
- 207: internal housing
- 208: first linear bearing surface
- 210: piston rod
- 211: second set of indentations
- 212: bearing surface
- 213: friction wheel assembly
- 214: drive member
- 215: longitudinal part
- 216: activation part
- 217: second linear bearing surface
- 218: grip surfaces
- 219: dispensing face
- 221: pawl means
- 222: cap
- 223: first set of indentations
- 227: friction wheel
- 228: carrier
- 229: pawl arms

## Claims

1. A drive mechanism for use in a drug delivery device, the drive mechanism comprising an assembly with a housing (203) having a proximal end and a distal end and a friction wheel (227), the drive mechanism further comprising:
a drive member (214) located within the said housing (203) such that the drive member (214) is movable longitudinally and is non-rotatable with respect to the housing (203);
a piston rod (210) that is non-rotatable with respect to the housing (203) and is adapted to operate through the housing (203) and to transfer a force in the longitudinal direction to the distal end of the drug delivery device (201);
the rotating friction wheel (227) releasably engaged with the piston rod (210) and engaged to a linear bearing surface (217) on the drive member and engaged to a linear bearing surface (208) on the housing, wherein,
a) when the drive member (214) moves proximally with respect to the housing (203) the friction wheel moves proximally with respect to the piston rod; and
b) when the drive member (214) moves distally the friction wheel (227) moves distally displacing the piston rod (210) towards the distal end of the device.

2. The drive mechanism of claim 1 wherein the friction wheel (227) is connected to a carrier plate (228) having pawl arms (229).

3. The drive mechanism according to claim 1 or 2, wherein the engagement between the piston rod (210) and the friction wheel (227) acts through an axle.

## Patentansprüche

1. Antriebsmechanismus zur Verwendung in einer Medikamenten-Verabreichungsvorrichtung, wobei der Antriebsmechanismus eine Anordnung mit einem Gehäuse (203), das ein proximales Ende und ein distales Ende hat, und einem Reibrad (227) umfasst, wobei der Antriebsmechanismus ferner Folgendes umfasst:
ein Antriebsglied (214), das in dem Gehäuse (203) angeordnet ist, so dass das Antriebsglied (214) in Längsrichtung bewegbar ist und bezüglich des Gehäuses (203) nicht drehbar ist,
eine Kolbenstange (210), die bezüglich des Gehäuses (203) nicht drehbar ist und geeignet ist, durch das Gehäuse (203) zu wirken und eine Kraft in der Längsrichtung an das distale Ende der Medikamenten-Verabreichungsvorrichtung (201) zu übertragen,
wobei das sich drehende Reibrad (227) lösbar mit der Kolbenstange (210) in Eingriff steht und mit einer linearen Lagerfläche (217) am Antriebsglied in Eingriff steht und mit einer linearen Lagerfläche (208) am Gehäuse in Eingriff steht,
wobei
a) sich das Reibrad proximal bezüglich der Kolbenstange bewegt, wenn sich das Antriebsglied (214) proximal bezüglich des Gehäuses (203) bewegt, und
b) sich das Reibrad (227) distal bewegt und die Kolbenstange (210) zum distalen Ende der Vorrichtung hin verschiebt, wenn sich das Antriebsglied (214) distal bewegt.

2. Antriebsmechanismus nach Anspruch 1, wobei das Reibrad (227) mit einer Trägerplatte (228) verbunden ist, die Klinkenarme (229) hat.

3. Antriebsmechanismus nach Anspruch 1 oder 2, wobei der Eingriff zwischen der Kolbenstange (210) und dem Reibrad (227) durch eine Achse wirkt.

## Revendications

1. Mécanisme d'entraînement destiné à une utilisation dans un dispositif d'administration de médicament, le mécanisme d'entraînement comprenant un ensemble avec un logement (203) comportant une extrémité proximale et une extrémité distale et une roue de friction (227), le mécanisme d'entraînement comprenant en outre :
un organe d'entraînement (214) situé à l'intérieur dudit logement (203) de telle sorte que l'organe d'entraînement (214) puisse être déplacé longitudinalement et ne puisse pas tourner par rapport au logement (203) ;
une tige de piston (210) qui ne peut pas tourner par rapport au logement (203) et est conçue pour fonctionner à travers le logement (203) et transférer une force dans la direction longitudinale à l'extrémité distale du dispositif d'administration de médicament (201) ;
la roue de friction rotative (227) interagissant de manière libérable avec la tige de piston (210) et interagissant avec une surface d'appui linéaire (217) sur l'organe d'entraînement et interagissant avec une surface d'appui linéaire (208) sur le logement,
dans lequel,
a) lorsque l'organe d'entraînement (214) se déplace dans la direction proximale par rapport au logement (203), la roue de friction se déplace dans la direction proximale par rapport à la tige de piston ; et
b) lorsque l'organe d'entraînement (214) se déplace dans la direction distale, la roue de friction (227) se déplace dans la direction distale en déplaçant la tige de piston (210) en direction de l'extrémité distale du dispositif.

2. Mécanisme d'entraînement selon la revendication 1, dans lequel la roue de friction (227) est raccordée à une plaque de support (228) comportant des bras formant cliquets (229).

3. Mécanisme d'entraînement selon la revendication 1 ou 2, dans lequel l'interaction entre la tige de piston (210) et la roue de friction (227) agit par le biais d'un axe.
